# EUROPEAN PATENT SPECIFICATION

(11) **EP 2 052 744 B1**
(45) Date of publication and mention of the grant of the patent: **22.12.2010**
(21) Application number: 08018681.0
(22) Date of filing: 24.10.2008
(51) Int. Cl.: A61L 2/08, B65B 55/08, B67C 7/00

(54) **Vessel sterilization apparatus**
Gefäßsterilisierungsvorrichtung
Appareil de stérilisation de récipient

(30) Priority: 26.10.2007 JP 2007278678
(43) Date of publication of application: 29.04.2009
(73) Proprietor: SHIBUYA KOGYO CO., LTD, Kanazawa-shi, Ishikawa-ken (JP)
(72) Inventor: Naka, Toshiaki, Kanazawa-shi, Ishikawa-ken (JP); Nishino, Yukinobu, Kanazawa-shi, Ishikawa-ken (JP); Nishi, Tokuo, Kanazawa-shi, Ishikawa-ken (JP)
(74) Representative: HOFFMANN EITLE

(56) References cited:
- EP-A- 1 849 746
- JP-A- 8 119 235
- JP-A- 2006 206 158
- JP-A- 2007 106 438

## Description

### BACKGROUND OF THE INVENTION

### Field of the Invention

The present invention relates to a vessel sterilization apparatus for performing sterilization of vessels by irradiating them with electron beam during conveyance thereof.

### Related Art

There has been known a technology of sterilizing an article such as vessel or film by being subjected to irradiation with electron beam (for example, refer to Patent Document 1: Japanese Patent Application Laid-open Publication No. 200661558 and Patent Document 2: Japanese Granted Patent Publication No. 1-17935).

The Patent Document 1 discloses an electron beam sterilization apparatus in which a scanning horn is disposed to irradiate a clean room filled with aseptic air with electron beam to thereby sterilize PET (polyethylene telephthalate) bottles during conveyance thereof in the clean room.

The Patent document 2 discloses a sterilizer for sterilizing a packaging material with electron beam, which performs sterilization of a packaging material by irradiating it with electron beam by using an electron beam irradiator within an electron beam sterilization chamber. In the sterilization chamber, there is provided a conduit for feeding N₂ gas, and the sterilization chamber is maintained in a positive pressure state by means of this N₂ gas than surroundings. The sterilization chamber is sterilized by cleaning, heating, H₂O₂-spraying and drying processes which are performed by means of pre-sterilization line, and thereafter, N₂ gas is supplied.

In the case of the sterilizer using the electron beam, sterilizing power is applied only to a portion which is irradiated with the electron beam, and the other area is not applied with any sterilizing power. Therefore, even in a case of preliminarily create a germicidal atmosphere in the inside of the sterilization chamber, if germ adhering to a vessel, before sterilization, introduced into the sterilization chamber floats in the chamber, the germ may again adhere to the vessel after the sterilization. Especially, in a case when the sterilization is performed during the conveyance of the vessel, the possibility of floating of the germ in the chamber increases because air may flow around the vessel, and hence, the possibility of re-adhering of the germ to the vessel will increase.

As mentioned above, even if the inside of the sterilization chamber is preliminarily made germ-free state, germ adhering to a vessel to be sterilized may not be sterilized even by irradiating the vessel with the electron beam, and hence, there poses a problem of less sterilization reliability to the sterilizer. In addition, in a case where a sterilizing gas such as H₂O₂ gas generated by evaporating H₂O₂ water is supplied in the sterilization chamber which is subjected to electron beam irradiation, the inside of the chamber is heated and the vessel is also heated excessively, and thereafter, when the electron beam irradiation is performed, the temperature inside the chamber is further increased, and there causes a fear of deforming the vessel by the excessive heat.

Furthermore, there has been proposed an invention, such as disclosed in Patent Document 3 (Japanese Patent Application Laid-open Publication No. 2007-106438), in which, beforehand the processing to the vessel by the electron beam irradiation, foreign material is not brought into a space which is subjected to the electron beam sterilization by removing the foreign material adhering to the vessel.

In the invention of this Patent Document 3, before the sterilization processing by the irradiation of a bottle vessel and a cap with the electron beam at the electron beam irradiation area in the clean room, in an ionized air jetting area formed on an upstream side of the electron beam irradiation area, a front end of a nozzle which jets ionization air inserted to a predetermined position of the bottle vessel, and by jetting the ionized air onto the inner surface of the bottle vessel, foreign material adhering to the inner surface of the bottle vessel is removed.

With the structure of the invention of the Patent Document 3, there is no fear of deforming the vessel by the excessive heating, but even if dust or germ adhering to the surface of the vessel is blown off by jetting the ionized air inside the vessel, there is a fear of floating of such light-weight impurities inside the processing chamber after once separation from the vessel and of re-adhering to the surface of the vessel.

EP 1 849 746 A1 is a European patent application which has been filed April 18, 2007 and published October 31, 2007. The EP 1 849 746 A1 describes an apparatus which sterilizes vessels carried by vessel holder means and conveyed within an aseptic chamber by irradiating the vessels with an electron beam from an electron beam irradiator. An injector which injects H₂O₂ gas is disposed within the aseptic chamber to maintain H₂O₂ gas atmosphere in the aseptic chamber.

### SUMMARY OF THE INVENTION

Therefore, one of the objects, for at least some embodiments of the present invention, is to provide a vessel sterilization apparatus capable of rendering a vessel supply area as sterilization area, in which electron beam sterilization is performed, preventing impurity such as germ from being brought into the sterilization area to thereby improve reliability of the electron beam sterilization, and possibly preventing a vessel from being deformed by excessive heating of the vessel.

According to the present invention, there is provided a vessel sterilization apparatus including a vessel conveying unit for conveying a vessel, and an electron beam irradiation unit for irradiating the vessel conveyed by the vessel conveying unit with an electron beam, the vessel sterilization apparatus further comprising:
a sterilization area in which the vessel is to be irradiated with the electron beam;
a first chamber surrounding the sterilization area so as to separate the sterilization area from externally thereof;
a supply area disposed at an upstream side of the sterilization area in a vessel conveying direction and provided with an opening for the vessel to pass through;
a second chamber surrounding the supply area so as to separate the supply area from externally thereof; and
a sterilizing medium supplying unit for supplying a sterilizing medium having a sterilizing property to the supply area so as to provide a germicidal gas atmosphere in an inside of the supply area, wherein the sterilizing medium is supplied, in use of the vessel sterilization apparatus, into a space of the supply area defined by a inner peripheral side wall surface of a vessel conveying path in the supply area, an outer peripheral side wall surface thereof and a bottom surface.

According to embodiments of the present invention, since the supply area for supplying the vessel in the sterilization area in which the vessel is sterilized by the irradiation with the electron beam is made as germicidal gas atmosphere, there is no fear of bringing any foreign material into the sterilization area, and hence, the reliability for sterilization by the electron beam can be improved. Moreover, it is not necessary to blow the germicidal gas into the sterilization area, the vessel is free from deformation by overheating.

Reference is now made to the following preferred examples.

The apparatus may further include an aseptic gas jetting unit for jetting aseptic gas into the inside of the vessel conveyed into the area in which the sterilizing medium is supplied to thereby substitute an inside air of the vessel with the aseptic gas.

it may be desired that the vessel is a PET bottle having a neck portion provided with a flange, the vessel is transferred with upper or lower portion of the flange being held, and the sterilizing medium supplying unit supplies the sterilizing medium toward the flange of the vessel.

The sterilizing medium supplying unit may be disposed in a space surrounded by vertical and lateral wall sections in the supply area.

It may be preferred that the sterilizing medium is a hydrogen peroxide gas, and the sterilizing medium is supplied in a mist state.

The nature and further characteristic features of the present invention will be made clearer from the following descriptions made with reference to the accompanying drawings and given by way of example.

### BRIEF DESCRIPTION OF THE DRAWINGS

In the accompanying drawings:

Fig. 1 is a plan view showing an entire structure of a vessel sterilization apparatus provided with an electron beam irradiation device according to one embodiment of the present invention;

Fig. 2 is a plan view of a rotary wheel provided with a sterilizing nozzle and an air jetting nozzle; and

Fig. 3 is an elevational section of the rotary wheel provided with the sterilizing nozzle and the air jetting nozzles.

### DESCRIPTION OF THE PREFERRED EMBODIMENT

Hereunder, an embodiment of the present invention will be described with reference to the accompanying drawings. Further, it is to be noted that terms "upper", "lower", "right", "left" and like terms are used herein with reference to the illustration on the accompanying drawings.

A vessel 2, which is subjected to sterilization by a vessel sterilization apparatus according to an embodiment of the present invention and which is filled up with a liquid, is a PET-bottle having a neck portion 2a at which a flange is formed. The PET-bottle is conveyed in a state that upper or lower portions of the flange of the neck portion 2a are supported.

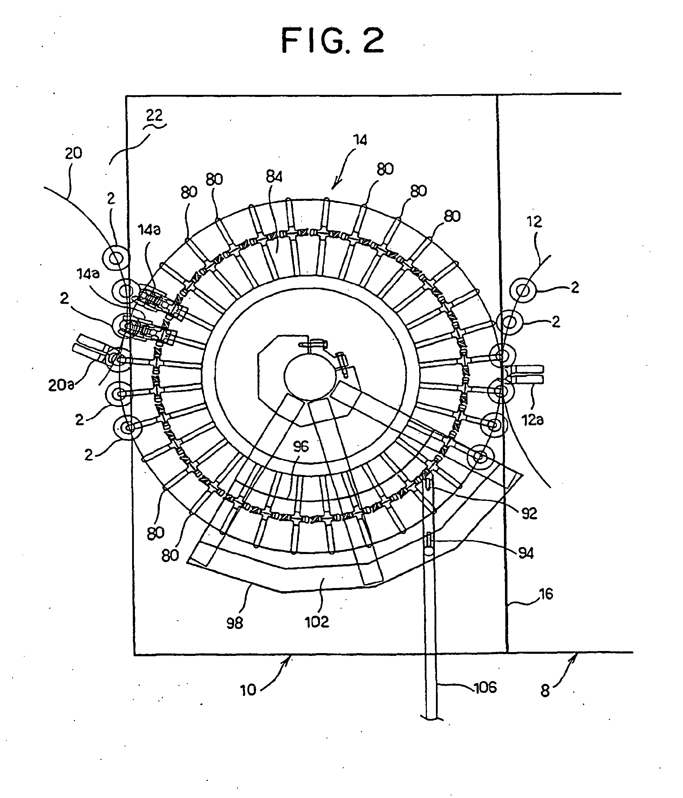

The vessels 2 are conveyed continuously by an air conveyer 4 with the lower surface sides of the flanges of the vessels 2 being held, respectively, into a supply chamber while separating at a predetermined interval by means of in-feed screw 6. The supply chamber includes two chamber sections including a first chamber section 8 and a second chamber section 10, in which rotary wheels including a first rotary wheel 12 and a second rotary wheel 14 provided with vessel holders 12a and 14a are arranged as shown in Fig. 2.

The vessels 2 conveyed into these supply chamber sections 8 and 10 are successively transferred, i.e., handed over, to the rotary wheels 12 and 14 in the supply chamber sections 8 and 10, respectively, and then conveyed while being rotated. An opening, not shown, through which the vessels 2 pass, is formed to a wall surface of the chamber, and the vessels 2 are conveyed into the first supply chamber 8 from the air conveyer 4, and on the other hand, an opening, not shown, through which the vessels 2 are handed over, is formed to a partition wall section 16 through which the vessel 2 is handed over from the first rotary wheel 12 to the second rotary wheel 14.

Close to the second supply chamber 10, there is provided a sterilization box 18 including a wall section made of lead for shielding X-ray and electron beam from leaking outside at the time when the vessel 2 is irradiated with the electron beam to sterilize the vessel 2. This sterilization box 18 constitutes a sterilizing area defined in the present patent claim.

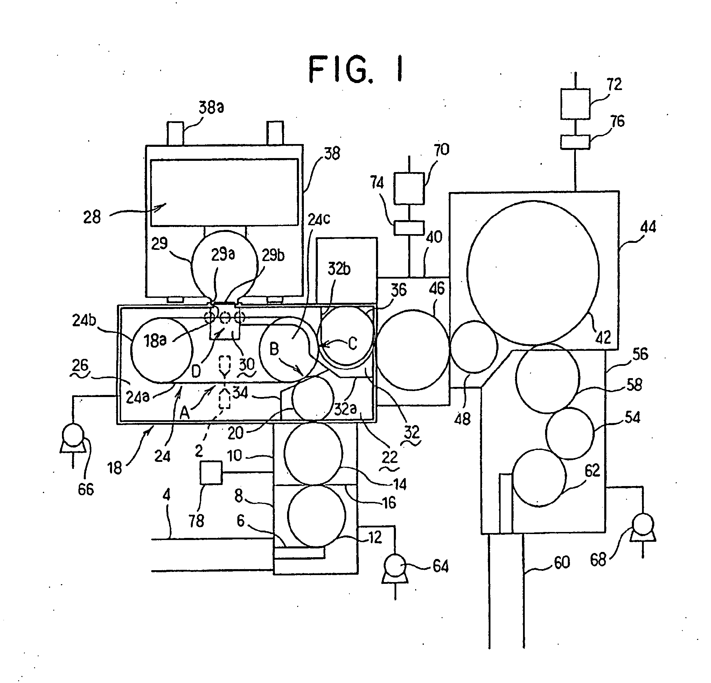

The inside of the sterilization box 18 is sectioned into: a supply chamber 22 on an inlet side at which a supply wheel 20 is disposed; a main chamber provided with a vessel conveyer 24 for conveying the vessel 2 received from the supply wheel 20 and inverting the vertical attitude of the vessel 2; an irradiation chamber 30 which is disposed on the front surface side of an electron beam irradiation device 28 and in which the conveyed vessel 2 is irradiated with the electron beam, and a discharge chamber 32 disposed so as to be continuous to the outlet side (right side in Fig. 1) and configured to transfer the vessel 2, to the downstream side, which is maintained in the sterilized state by the irradiation of the electron beam.

The vessel 2 is transferred to a vessel holder 20a of the supply wheel 20 from a vessel holder 14a of the second rotary wheel 14a as shown in Fig. 2, and thereafter, the vessel 2 is handed over to the vessel conveyer 24. A portion over which the vessel 1 is handed to supply wheel 20 in the supply chamber 22 from the rotary wheel 14 of the second supply chamber 10 is formed with an opening, not shown, through which the vessel 2 can pass.

The supply wheel 20 transferring the vessel 2 from the second rotary wheel 14 transfers the vessel 2 to the vessel conveyer 24 disposed in the main chamber 26. Another opening, not shown, through which the vessel 2 can be handed over, is also formed to a partition wall 34 formed between the supply chamber 22 and the main chamber 26.

The vessel conveyer 24 disposed in the main chamber 26 is provided with a vessel holding belt 24a formed as an endless vessel conveyer provided with continuously located vessel grippers as holders for holding a plurality of vessels 2 and two sprockets 24b, 24c as conveying rotary member for circularly conveying the vessel grippers around which the endless vessel holding belt 24 stretched.

Further, in this embodiment, an area composed of the main chamber 26 and the irradiation chamber 30 corresponds to the sterilization area defined in present patent claim, and an area composed of the first supply chamber 8, the second supply chamber 10, and the supply chamber 22 corresponds to the supply area defined in the present patent claim.

Each of the vessel grippers includes a pair of vertical vessel holders so that two vessels 2 are simultaneously held and conveyed, and rotate by 180 degrees around an axis extending along the conveying direction. The vessel conveyer 24 includes a straight path conveying straightly the vessel gripper between the sprockets 24b and 24c and a circumferential path conveying circumferentially the vessel gripper around the sprockets 24b and 24c, and an inverting position A is provided in the straight path between the sprockets 24b and 24c at the which the vessel 2 is turned by one turn during the passing of the circumferential path so as to change the vertical position of the vessel by 180 degrees.

A vessel supply position B is provided on the downstream side of the gripper conveying direction in the circumferential path of one of the sprockets 24c (right side sprocket in Fig. 1), and on the upstream side thereof, a discharge position C is provided. According to such arrangement, one vessel 2 is conveyed by two turns with being held at the supply position B by one vessel conveyer, and during this turning, the attitude of the vessel 2 is inverted twice vertically to take the original position, and thereafter, the vessel 2 is handed over to a transfer wheel 36 disposed in the discharge chamber 32 at the discharge position C.

The electron beam irradiation device 28 is disposed in adjacent to the lead-made sterilization box 18. The electron beam irradiation device 28 includes an irradiation unit 29 for irradiating the vessel 2 with the electron beam, and disposed on a rest table 38. Since the electron beam irradiation device 28 has structures and the functions which are generally known, detailed explanation and illustration thereof are omitted herein. However, the electron beam irradiation device is a device for generating thermoelectron by heating filament in vacuum condition in the irradiation unit 29, accelerating the electron by high voltage to create high speed electron beam, taking out into atmosphere through window foil 29b made of Ti or like attached to an irradiation window 29a, and then performing a sterilization treatment or like by irradiation of the electron beam to an object (i.e., vessel 2).

In this embodiment, the rest table 38 on which the electron beam irradiation device 28 is placed is movable along rails 38a so as to approach the sterilization box 18 and separate therefrom. When the vessel sterilization device is operated, the rest table 38 is moved closely to the sterilization box 18, the irradiation window 29a of the irradiation unit 29 accords with the opening 18a formed to the wall surface of the sterilization box 18, and then, the sterilization box 18 and the irradiation unit 29 are connected together.

Inside the sterilization box 18, there is disposed the irradiation chamber 30 so as to surround the opening 18a to which the irradiation unit 29 is connected. The straight path between the sprockets 24b and 24c of the vessel conveyer 24 penetrates the irradiation chamber 30, and an irradiation position D is set the penetrating portion of the straight path. Two vessels 2 held by the vessel grippers pass through the irradiation chamber 30 in the vertically perpendicular attitude, and during the passing, the vessels 2 are irradiated with the electron beam from the electron beam irradiation device 28 so as to sterilize the vessel.

An opening, not shown, is formed to wall surfaces on the inlet and outlet sides of the irradiation chamber 30 so that vertical two vessels 2 held by the vessel grippers can pass through the opening, and the discharge chamber 32 is formed to be continuous to the outlet side wall surface of the irradiation chamber 30. One of the sprockets 24c (right side one in Fig. 1) intrudes into the discharge chamber 32, and is held by the vessel gripper therein. The vessel 2 subjected to the irradiation of the electron beam twice at the upper and lower positions is handed over to the transfer wheel 36 disposed in the discharge chamber 32 from the vessel holder positioned below the vessel gripper in the discharge chamber 32.

This discharge chamber 32 is surrounded and defined by: a partition wall section 32a sectioning, without disturbing the rotation of the sprocket 24c, the conveying path of the vessel conveyer 24 between the opening on the outlet side of the irradiation chamber 30 and the transfer wheel 36: a partition wall section 32b opposing to the partitioning wall section 32a and sectioning the transfer wheel 36 from the vertical space; and floor and ceiling sections of the sterilization box 18. Chambers succeeding to this discharge chamber 32 are ones for treating or processing the vessels 2 sterilized by the irradiation of the electron beam, so that the germ-free (germicidal) state can be maintained.

An intermediate chamber 40 is arranged in adjacent to the discharge chamber 32 disposed on the most downstream side in the sterilization box 18. A chamber 44 in which a filler 42 is accommodated is disposed on the downstream side of this intermediate chamber 40, and a rotary wheel (neck wheel) 46 provided with a vessel holder is also arranged in the intermediate chamber 40. The neck wheel 46 serves to transfer the vessel 2, after the conveyance thereof, received from the transfer wheel 36 to a supply wheel 48 disposed in the chamber 44 in which the filler 42 is mounted.

The vessel 2 is supplied to the filler 42 after the vessel 2 has been handed over to the supply wheel 48 disposed on the inlet side of the chamber 44. The filler 42 receiving the vessel 2 from the supply wheel 48 is filled up with content such as liquid during the conveyance, while rotating, thereof in the vessel holding state.

The vessel 2 filled up with the content is conveyed into a chamber 56 in which a capper 54 is disposed adjacently to the chamber 44 in which the filler 42 is disposed. An intermediate wheel 58 is arranged on the inlet side of the chamber 56 for the capper 54 for transferring the vessel 2 from the filler 42 to the capper 54. Furthermore, on the downstream side of the capper 54, is provided a discharge wheel 62 for handing over the vessel 2 with a cap to a discharge conveyer 60.

According to the vessel sterilization apparatus of the structure mentioned above, the vessel 2 conveyed by means of air conveyer 4 is subjected to the irradiation with the electron beam from the electron beam irradiation device 28 in the sterilization box 18 to thereby sterilize the vessel 2. Thereafter, the vessel 2 is filled up with the inner content by means of filler 42 and the capping operation is effected by the capper 54, and then, discharged by the discharge conveyer 60 so as to be subjected to the succeeding process.

Further, the openings, through which the vessel 2 can pass, are formed respectively to the wall surface portions corresponding to the positions (1) at which the vessel 2 is handed over to the rotary wheel 46 of the intermediate chamber 40 from the transfer wheel 36 disposed in the discharge chamber 32 in the sterilization box 18, (2) at which the vessel 2 is handed over to the supply wheel 48 in the chamber 44 in which the filler is disposed from the rotary wheel 46 in the intermediate chamber 40 and (3) at which the vessel is handed over from the filler 42 to the intermediate wheel 58 in the chamber in which the capper 54 is disposed.

The respective openings of the sterilization box 18 are covered by shutters, not shown, which are closed at a time when the communicating other chamber is washed to thereby prevent water drops or moisture content from flowing inside.

Furthermore, discharge blowers 64, 66 and 68 are connected to the first supply chamber 8, the main chamber 26 of the lead-made sterilization box 18, and the chamber 56 provided with the capper 54, respectively, to thereby discharge the air in the respective chambers 8, 26 and 56. Further, pressurized air supply members 70 and 72 are connected, through filters 74 and 76, to the intermediate chamber 40 and the chamber 44 in which the filler 42 is disposed so as to supply aseptic air into the chambers 40 and 44, respectively.

The discharge blowers 64, 66 and 68, the pressurized air supply members 70 and 72, and control devices, not shown, for controlling air discharge amount and the air supply amount constitute a pressure control unit, by which the pressures in the respective chambers 8, 10, 22, 26, 32, 40, 44 and 56 are controlled.

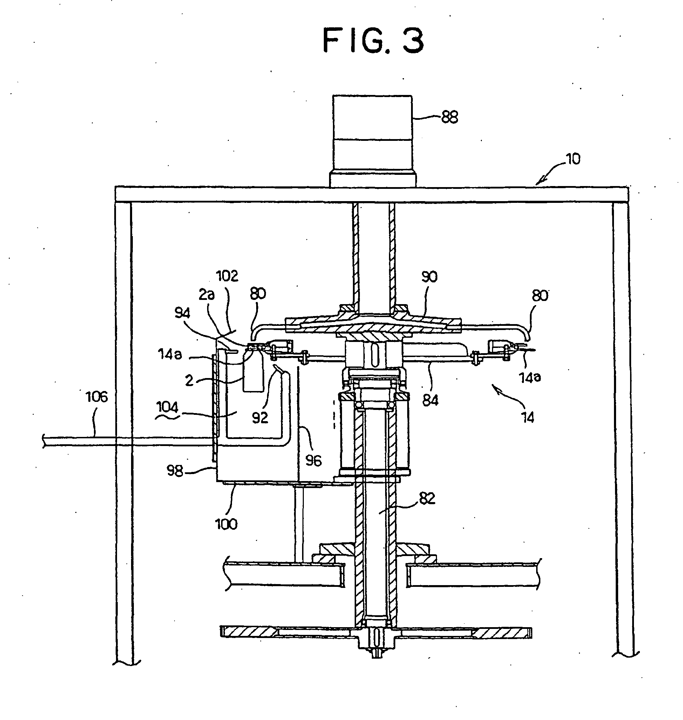

Still furthermore, in the vessel sterilization apparatus according to the present embodiment, a supply member 78 for supplying a sterilizing medium such as germicidal gas in this embodiment is connected to the second supply chamber 10 so as to create a germicidal gas atmosphere in the second supply chamber (supply area) and to replace the inside of the vessel introduced into this chamber 10 with the aseptic air. These structures will be explained hereunder with reference to Figs. 2 and 3.

An aseptic air jetting nozzle 80 for blowing aseptic air into the vessel 2 now in conveyance is provided for the second rotary wheel 14 disposed in the second supply chamber 10. The second rotary wheel 14 includes a star wheel 84 rotated by a rotating shaft 82, and the vessel supporting members 14a for holding the neck portions 2a of the vessels 2 are disposed in the circumferential direction at equal interval in the outer peripheral portion of the star wheel 84.

The aseptic air jetting nozzles 80 are arranged in correspondence to the vessel supporting members 14a, respectively. The aseptic air jetting nozzles 80 are arranged so as to oppose to mouth portions of the vessels 2 held by the vessel supporting members 14. Aseptic air introduced from outside through a rotary joint 88 is distributed to the respective aseptic air jetting nozzles 80 by means of manifold 90. Although in this embodiment, such aseptic air is always jetted through the respective aseptic air jetting nozzles 80 into the vessels 2 during the operation of the apparatus, the jetting of the aseptic air may be on/off controlled.

Sterilizing nozzles 92 and 94 are disposed in the second supply chamber 10 to create the germicidal gas atmosphere in the chamber 10. The sterilizing nozzles 92 and 94 are arranged on inner and outer peripheral sides of the vessel conveying path formed by the star wheel 84 so as to jet gas such as hydrogen peroxide gas (H₂O₂), in this embodiment, toward the flanged portion of the vessel 2 during the conveyance thereof. The sterilizing nozzles 92 and 94 are arranged in a space defined by an inner peripheral side wall surface 96 of vessel conveying path, an outer peripheral side wall surface 98 thereof, a bottom surface 100 and a hood disposed to an upper portion of the outer peripheral side. The sterilizing nozzles 92 and 94 serve to jet the hydrogen peroxide gas supplied through a hydrogen peroxide gas supply path from the sterilizing gas supply member 106 disposed externally to portions near the flanged portions of the vessels 2 having been conveyed in the space 104. This space 104 occupies 1/4 of the entire vessel conveying path, and the hydrogen peroxide gas jetted toward the vessel 2 in the space 104 is diffused entirely inside the second supply chamber 10, thus creating the hydrogen peroxide gas atmosphere in the chamber 10 formed as supply area.

Further, in this embodiment, although the hydrogen peroxide gas is introduced into the second supply chamber 10, in an alternation, it is always not necessary to supply it in gaseous state, and the hydrogen peroxide may be supplied in mist state, which is then gasified in the chamber 10. That is, the atmosphere to be supplied to the second supply chamber 10 is a gas, but it may take gaseous state or mist state at a time of being supplied in the second supply chamber.

Furthermore, in this embodiment, although the vessel 2 is externally conveyed into the second supply chamber 10 and sterilized therein after making the inside space of the second supply chamber to the hydrogen peroxide gas atmosphere state, the sterilized state may be created in either one of the first supply chamber 8 or the supply chamber 22 without limiting to the second supply chamber 10. At any rate, it may be preferable to making at least a portion in the supply area disposed upstream side of the sterilizing area, i.e., an area separated from external portions, to the sterilizing atmosphere and to transfer the vessel into this area to thereby sterilize impurities such as germ adhering to the vessel 2.

The vessel sterilization apparatus of the structure mentioned above will function as follows.

The vessel which is sterilized by this vessel sterilization apparatus and filled up with liquid or like is a PET bottle, which is conveyed by the air conveyer 4 by blowing air from the rear side in a state that the lower side of the flange formed to the neck portion 2a is held.

The vessels 2 conveyed by the air conveyer 4 are guided into the first supply chamber 8, in which the vessels 2 are separated by the in-feed screw 6 at a predetermined interval and then handed over to the vessel holder 12a of the first rotary wheel 12. The vessel 2, after being rotated and transferred by the rotary wheel 12, is handed over to the second rotary wheel 14 disposed in the second supply chamber 10.

Then, the vessels 2 are rotated and transferred by the second rotary wheel 14 in the state that the lower sides of the flanges of the vessels 2 are held by the vessel holder 14a provided for the star wheel 84. Above the mouth portions of the vessels held by the vessel holder 14a, the aseptic air jetting nozzles 80 are respectively arranged so as to always jet the aseptic air into the vessels 2.

As mentioned above, by flowing the aseptic air into the vessel 2, the air inside the vessel 2 is substituted with the aseptic air. Furthermore, in the second supply chamber 10, the hydrogen peroxide gas jetting nozzles 92 and 94 are arranged so as to jet gas obtained by gasifying the hydrogen peroxide solution and to fill the inside of the second supply chamber 10, forming a portion of the supply area, with the sterilizing hydrogen peroxide gas, thus sterilizing the surfaces of the vessels 2 conveyed in the second supply chamber 10.

When the vessels 2 held by the vessel holder 14a of the star wheel 84 are rotated and transferred, advance in the space surrounded by the inner and outer wall surfaces 96, 98, the bottom surface 100 and the upper hood 102 and reach the position of the hydrogen peroxide gas jetting nozzle positions, the hydrogen peroxide gas is jetted directly toward the portions near the flanges of the neck portions 2a of the vessels 2 by the hydrogen peroxide gas jetting nozzles 92 and 94 located inner and outer peripheral sides of the conveying path. Thereafter, the vessels 2 are sterilized by being irradiated with the electron beam. However, because the portion of the vessel 2 held by the gripper of the vessel conveyer 24 may become a dead portion and not completely be sterilized, the hydrogen peroxide gas is directly jetted to the portion near the flange of the neck portion 2a of the vessel 2 by the hydrogen peroxide gas jetting nozzles 92 and 94.

Furthermore, at the time when the air inside the vessel 2 is replaced with the aseptic air jetted by the aseptic air jetting nozzle 80, impurities such as germ adhering to the inner surface of the vessel or existing inside the vessel 2 are discharged outward, and since the inner space of the second supply chamber 10 becomes hydrogen peroxide gas atmosphere, such impurities can be sterilized by the hydrogen peroxide gas. Accordingly, the re-adhesion of the impurity to the surface of the vessel 2 can be prevented and the impurity never be brought into the next sterilization area such as main chamber 26 or irradiation chamber 30.

Subsequently, the vessel 2 is handed over to the supply wheel 20 disposed in the supply chamber 22 of the lead-made sterilization box 18 from the second rotary wheel 14, rotated and transferred in the state held by the vessel holder 20a of the supply wheel 20, and then, is handed over to the gripper of the vessel conveyer 24. The gripper includes the vertically arranged two vessel holders, and the vessel 2 held by the lower-side holder is moved upward by the inverted turning of the gripper and takes the inverted state. The vessel gripper in the inverted attitude is moved, while rotating around the sprocket, into the irradiation chamber 30. The vessel 2 is therein irradiated with the electron beam from the electron beam irradiation device 28, and the conveying vessel 2 is irradiated with the electron beam during the conveyance at a time passing in front of the window foil 29b of the irradiation unit 29, thus being sterilized.

The vessel 2 passing through the irradiation chamber 30 after being irradiated with the electron beam therein moves inside the discharge chamber 32, rotates and moves around the sprocket 24c, and then again returns to the transfer position from the supply wheel 20. The vessel 2 which was transferred before by the lower side vessel holder from this supply wheel 20 has been inverted and moved upward, and accordingly, the other vessel holder positioned below receives the next vessel 2 from the supply wheel 20. Thereafter, the gripper is again inverted so as to be upside down to thereby move downward the vessel 2, the upper portion of which was irradiated with the electron beam during the former movement, and the surface not subjected to the irradiation of the electron beam is directed toward the outside of the rotationally moving direction of the vessel conveyer 24.

When the vessel 2 is again moved into the irradiation chamber 30, the vessel 2 which had already been subjected to the first irradiation of the electron beam is subjected to the second irradiation of the electron beam from the side opposite to the first irradiation side, thus the inner and outer surfaces of the vessel 2 being irradiated with the electron beam.

The vessel 2 irradiated with the electron beam and entirely sterilized by the second irradiation of the electron beam in the irradiation chamber 30 is handed over to the transfer wheel 36 in the discharge chamber 32, and then further handed over to the neck wheel 46 in the succeeding intermediate chamber 40. The vessel 2 is thereafter discharged from the lead-made sterilization box 18.

The vessel 2 is further handed over to the supply wheel 48 disposed in the chamber 44, in which the filler 42 is arranged, from the neck wheel 46 disposed in the intermediate chamber 40. Thereafter, the vessel 2 is transferred to the filler 42 from the supply wheel 48. The vessel 2 filled up with the inner content during the rotating and conveying by the filler 42 is taken out from the filler 42 by the intermediate wheel 58 and transferred into the chamber 56 in which the capper 54 is arranged.

After the transferring of the vessel 2 to the capper 54 from the intermediate wheel 58 and the capping operation by the capper 54, the capped vessel 2 is discharged on the discharge conveyer 60 through the discharge wheel 62 to be subjected to the subsequent procedure.

It is further to be noted that the present invention is not limited to the described embodiment and many other changes and modifications may be made without departing from the scopes of the appended claims.

For example, in the described embodiment, although the irradiation chamber 30 is provided, the irradiation chamber 30 may be eliminated. In addition, there may be eliminated the structure in which the vessels are sterilized by directly jetting the sterilizing medium by the sterilizing nozzles 92, 94, and conduits or like for supplying the sterilizing medium may be provided on the chamber wall surfaces, alternatively.

## Claims

1. A vessel sterilization apparatus including a vessel conveying unit (24) for conveying a vessel (2), and an electron beam irradiation unit (28) for irradiating the vessel (2) conveyed by the vessel conveying unit (24) with an electron beam, the vessel sterilization apparatus further comprising:
a sterilization area (26,30) in which the vessel (2) is to be irradiated with the electron beam;
a first chamber surrounding the sterilization area (26,30) so as to separate the sterilization area (26,30) from externally thereof;
a supply area (8, 10, 22) disposed at an upstream side of the sterilization area (26,30) in a vessel conveying direction and provided with an opening for the vessel (2) to pass through;
a second chamber surrounding the supply area (8, 10, 22) so as to separate the supply area (8, 10, 22) from externally thereof; and
a sterilizing medium supplying unit (92,94,106) for supplying a sterilizing medium having a sterilizing property to the supply area (8, 10, 22) so as to provide a germicidal gas atmosphere in an inside of the supply area (8,10,22), wherein the sterilizing medium is supplied, in use of the vessel sterilization apparatus, into a space (104) of the supply area (8,10,22) defined by a inner peripheral side wall surface (96) of a vessel conveying path in the supply area, an outer peripheral side wall surface (98) thereof and a bottom surface (100).

2. The vessel sterilization apparatus according to claim 1, further comprising an aseptic gas jetting unit (80) for jetting aseptic gas into the inside of the vessel conveyed (2) into the supply area in which the sterilizing medium is supplied to thereby substitute an inside air of the vessel (2) with the aseptic gas.

3. The vessel sterilization apparatus according to claim 1 or 2, wherein the vessel (2) is a polyethylene telephthalate (PET) bottle having a neck portion (2a) provided with a flange, the vessel (2) is transferred with an upper or lower portion of the flange being held, and the sterilizing medium supplying unit (92,94,106) is arranged to supply the sterilizing medium toward the flange of the vessel.

4. The vessel sterilization apparatus according to any one of the preceding claims, wherein the sterilizing medium is a hydrogen peroxide gas.

5. The vessel sterilization apparatus according to any one of the preceding claims, wherein the sterilizing medium is arranged to be supplied in a mist state.

## Patentansprüche

1. Vorrichtung zum Sterilisieren von Gefäßen, die eine Gefäßbeförderungseinheit (24), um ein Gefäß (2) zu befördern, und eine Elektronenstrahlbestrahlungseinheit (28), um das Gefäß (2), das durch die Gefäßbeförderungseinheit (24) befördert wird, mit einem Elektronenstrahl zu bestrahlen, aufweist, wobei die Vorrichtung zum Sterilisieren von Gefäßen weiter aufweist:
einen Sterilisationsbereich (26, 30), in dem das Gefäß (2) mit dem Elektronenstrahl bestrahlt werden soll,
eine erste Kammer, welche den Sterilisationsbereich (26, 30) umgibt, um den Sterilisationsbereich (26, 30) von dem Äußeren davon abzutrennen,
eine Zuführfläche (8, 10, 22), die an einer in der Gefäßzuführrichtung vorgelagerten Seite des Sterilisationsbereichs (26, 30) vorgesehen ist und mit einer Öffnung, um das Gefäß (2) hindurchzulassen, versehen ist,
eine zweite Kammer, welche die Zuführfläche (8, 10, 22) umgibt, um die Zuführfläche (8, 10, 22) von dem Äußeren davon abzutrennen, und
eine Sterilisationsmittelzuführeinheit (92, 94, 106), um ein Sterilisierungsmittel, das eine Sterilisierungseigenschaft aufweist, der Zuführfläche (8, 10, 22) zuzuführen, um eine keimtötende Gasatmosphäre in einem Inneren der Zuführfläche (8, 10, 22) zu schaffen, wobei das Sterilisierungsmittel beim Betrieb der Vorrichtung zum Sterilisieren von Gefäßen in einen Raum (104) der Zuführfläche (8, 10, 22) zugeführt wird, der durch eine innere Seitenwandoberfläche (96) eines Gefäßzuführpfads in der Zuführfläche, eine äußere Seitenwandoberfläche (98) davon und eine untere Oberfläche (100) definiert wird.

2. Vorrichtung zum Sterilisieren von Gefäßen nach Anspruch 1, die weiter eine aseptische Gasstrahleinheit (80) aufweist, um aseptisches Gas in das Innere des Gefäßes (2), das in die Zuführfläche zugeführt wird, in die das Steriliserungsmittel zugeführt wird, um **dadurch** eine innere Luft des Gefäßes (2) mit dem aseptischen Gas zu ersetzen, einzustrahlen.

3. Vorrichtung zum Sterilisieren von Gefäßen nach Anspruch 1 oder 2, wobei das Gefäß (2) eine Polyethylentelephthalat (PET) Flasche mit einem Halsabschnitt (2a), der mit einem Flansch versehen ist, ist, wobei das Gefäß (2) befördert wird, während ein oberer oder unterer Abschnitt des Flansches gehalten wird, und die Steriliserungsmittelzuführeinheit (92, 94, 106) so vorgesehen ist, dass sie das Sterilisierungsmittel zu dem Flansch des Gefäßes hin zuführt.

4. Vorrichtung zum Sterilisieren von Gefäßen nach einem der vorhergehenden Ansprüche, wobei das Sterilisierungsmittel ein Wasserstoffperoxidgas ist.

5. Vorrichtung zum Sterilisieren von Gefäßen nach einem der vorhergehenden Ansprüche, wobei das Sterilisierungsmittel dafür vorgesehen ist, in einem nebelartigen Zustand zugeführt zu werden.

## Revendications

1. Appareil de stérilisation de récipient, comprenant une unité de convoyage de récipient (24) pour convoyer un récipient (2) et une unité d'irradiation à faisceau d'électron (28) pour irradier le récipient (2) convoyé par l'unité de convoyage du récipient (24) avec un faisceau d'électron, l'appareil de stérilisation de récipient comprenant en outre :
une zone de stérilisation (26, 30) dans laquelle le récipient (2) doit être irradié avec le faisceau d'électron ;
une première chambre entourant la zone de stérilisation (26, 30) de façon à séparer la zone de stérilisation (26, 30) de l'extérieur de celle-ci ;
une zone d'apport (8, 10, 22) disposée d'un côté en amont de la zone de stérilisation (26, 30) dans une direction de convoyage du récipient et dotée d'une ouverture pour que le récipient (2) y passe au travers ;
une deuxième chambre entourant la zone d'apport (8, 10, 22) de façon à séparer la zone d'apport (8, 10, 22) de l'extérieur de celle-ci ; et
une unité d'apport de milieu stérilisant (92, 94, 106) pour fournir un milieu stérilisant ayant une propriété stérilisante à la zone d'apport (8, 10, 22) de façon à assurer une atmosphère de gaz germicide dans un intérieur de la zone d'apport (8, 10, 22), le milieu stérilisant étant fourni, lors de l'utilisation de l'appareil de stérilisation de récipient, dans un espace (104) de la zone d'apport (8, 10, 22) défini par une surface de paroi latérale périphérique interne (96) d'une voie de convoyage du récipient dans la zone d'apport, une surface de paroi latérale périphérique externe (98) de celle-ci et une surface de fond (100).

2. Appareil de stérilisation de récipient selon la revendication 1, comprenant en outre une unité d'injection de gaz aseptique (80) pour injecter un gaz aseptique dans l'intérieur du récipient convoyé (2) dans la zone d'apport dans laquelle le milieu stérilisant est fourni pour substituer ainsi un air intérieur du récipient (2) par le gaz aseptique.

3. Appareil de stérilisation de récipient selon la revendication 1 ou 2, dans lequel, le récipient (2) est une bouteille de polyéthylène téréphtalate (PET) ayant une partie de col (2a) dotée d'un rebord, le récipient (2) étant transféré avec une partie supérieure ou inférieure du rebord qui est retenue et l'unité d'apport de milieu stérilisant (92, 94, 106) est conçue de façon à apporter le milieu stérilisant vers le rebord du récipient.

4. Appareil de stérilisation de récipient selon l'une quelconque des revendications précédentes, dans lequel le milieu stérilisant est un gaz de peroxyde d'hydrogène.

5. Appareil de stérilisation de récipient selon l'une quelconque des revendications précédentes, dans lequel le milieu stérilisant est conçu pour être fourni à l'état d'une brume.
